## Europäisches Patentamt

## European Patent Office

## Office européen des brevets

(11) Veröffentlichungsnummer: **0 222 971**
A1

(12) # EUROPÄISCHE PATENTANMELDUNG

(21) Anmeldenummer: 86109433.2

(22) Anmeldetag: 10.07.86

(51) Int. Cl.⁴: **B 25 B 23/08**, A 61 B 17/56

(30) Priorität: 07.11.85 DE 3539502

(43) Veröffentlichungstag der Anmeldung: 27.05.87
Patentblatt 87/22

(84) Benannte Vertragsstaaten: AT BE CH DE FR GB IT LI SE

(71) Anmelder: Oswald Leibinger GmbH,
Josef-Lang-Strasse 22, D-7202 Mühlheim-Stetten (DE)

(72) Erfinder: Leibinger, Karl, Schauinsland-Strasse 17,
D-7200 Ruttlingen-Möhringen (DE)
Erfinder: Leibinger, Franz, Nelkenstrasse 24,
D-7202 Mühlheim-Stetten (DE)

(74) Vertreter: Patentanwälte Dipl.-Ing. Klaus Westphal Dr.
rer. nat. Bernd Mussgnug Dr. rer.nat. Otto Buchner,
Waldstrasse 33, D-7730 VS-Villingen (DE)

(54) Schraubendreher, insbesondere für chirurgische Zwecke.

(57) Ein Schraubendreher, insbesondere für Knochenschrauben, wird so ausgebildet, daß er sowohl zum Entnehmen einer Schraube aus einem Sortiment als auch zum vollständigen Einschrauben und Festziehen der Schraube in einen Knochen, ggf. durch die Öffnung einer am Knochen zu befestigenden Knochenplatte hindurch, ohne Zuhilfenahme eines zweiten Werkzeugs geeignet ist, indem die zur Festlegung der Schraube während des Entnehmens und Andrehens notwendigen Greiforgane nach dem Andrehen zurückgezogen werden können. Durch Zweiteilung des Handgriffes wird eine bequeme und gleichzeitig sehr sensible Betätigbarkeit der Schraube erzielt. Durch eine lösbare Kupplung zwischen Werkzeugschaft und Handgriff läßt sich eine vollständige Sterilisierbarkeit des Schraubendrehers erzielen.

Dipl. Ing. Klaus Westphal

Dr. rer. nat. Bernd Mussgnug

Dr. rer. nat. Otto Buchner

PATENTANWÄLTE
European Patent Attorneys

Waldstrasse 33

D-7730 VS-VILLINGEN

Flossmannstrasse 30a

D-8000 MÜNCHEN 60

Telefon 07721-56007
Telegr. Westbuch Villingen
Telex 5213177 webu d

Telefon 089-832446
Telegr. Westbuch München
Telex 5213177 webu d
Telecop. 089-8344618
(CCITT 2) attention webu

0222971

- 1 -

Patentanmeldung P 35 39 502.8-15

- Oskar Leibinger GmbH -

u.Z.: 971.31
-EP

Schraubendreher, insbesondere für chirurgische Zwecke

Die Erfindung betrifft einen Schraubendreher nach dem Oberbegriff des Anspruchs 1.

Ein derartiger Schraubendreher ist aus der US-PS 3 498 361 bekannt. Der bekannte Schraubendreher ist zwar für chirurgische und andere diffizile Verschraubungsvorgänge zweckmäßig einsetzbar. Es ist jedoch nicht möglich, mit dem bekannten Schraubendreher Schrauben mit verschiedenem Kopf, beispielsweise mit Längsschlitz, Kreuzschlitz, Phillips-Kopf, Imbus-Kopf und dgl. zu drehen, sondern es ist für jede Schraubenart ein anderer Schraubendreher zu verwenden. Für weitere, insbesondere chirurgische, Arbeitsgänge sind noch zahlreiche weitere Werkzeuge erforderlich, so daß für eine Operation sehr viele verschiedene Werkzeuge vorhanden sein müssen. Außerdem ist die Sterilisierung des bekannten Schraubendrehers wegen der zahlreichen ineinander gefügten und gegeneinander verschiebbaren Teile nicht ganz einfach. Auch eine einwandfreie und feinfühlige Betätigbarkeit des bekannten Schraubendrehers unter schwierigen Bedingungen, beispielsweise mit Operationshandschuh, ist nicht gewährleistet.

Durch die Erfindung soll daher ein Schraubendreher der eingangs genannten Art so verbessert werden, daß er einerseits für alle Schraubenarten und auch andere, insbesondere beim Operieren vor-

Postscheckkonto Karlsruhe 76979-754 Bankkonto: Deutsche Bank AG Villingen (BLZ 69470039) 146332 kommende Arbeitsgänge verwendbar wird, wobei insbesondere die Sterilisierung seiner Teile und seine Betätigung unter schwierigen Bedingungen wesentlich erleichtert wird.

Diese Aufgabe wird erfindungsgemäß durch die kennzeichnenden Merkmale des Anspruchs 1 gelöst.

Durch die auswechselbare Halterung des das Werkzeug tragenden Schaftes am vorderen Griffteil kann in sehr einfacher Weise ein Schaft mit einem jeweils benötigten Werkzeug eingesetzt und schnell ausgewechselt werden. Auch sind für verschiedene Operationstechniken verschieden lang bemessene Schäfte mit u. U. verschieden dimensionierten und gestalteten Arbeitsenden erforderlich, so daß es durch die Auswechselbarkeit des Schaftes ohne weiteres möglich ist, Werkzeuge mit unterschiedlich langen Schäften und verschieden dimensionierten und gestalteten Arbeitsenden einzusetzen. Die Demontierbarkeit des erfindungsgemäßen Schraubendrehers ergibt überdies eine sehr gute Sterilisierbarkeit, was für die Anwendung bei einer Operation Voraussetzung ist. Die zweiteilige Ausgestaltung des Handgriffes ermöglicht es insbesondere, den rückwärtigen Griffteil sicher und ermüdungsfrei in der Hand zu halten und den vorderen, dagegen drehbaren Griffteil mit Daumen und Zeigefinger feinfühlig zu drehen, ohne Reibungskräfte zwischen der Hand bzw. dem Handschuh und dem festliegenden rückwärtigen Griffteil hervorzurufen. Dadurch läßt sich der Schraubendreher mit Fingerspitzengefühl drehen und das auf die Schraube zu übertragende Drehmoment wird begrenzt, so daß die Beschädigung einer Schraube durch Überdrehen weitgehend vermieden wird. Bei dem bekannten Schraubendreher konnte die Schraube mit der gesamten Kraft des Hand- oder Armgelenkes angezogen werden, was häufig zum Ausreißen der Schraube aus dem Knochen oder zur Beschädigung bzw. zum Abreißen des Schraubenkopfes führte. Außerdem ist bei Verwendung des bekannten Schraubendrehers nicht selten der Handschuh des Operateurs beschädigt worden, was sowohl die Infektionsgefahr erhöhte als auch einen sehr zeitraubenden Handschuhwechsel erforderlich machte.

- 3 -

Im Hinblick auf Festigkeit und Sterilisierbarkeit werden als Materialien für das Werkzeug, die Spannhülse und die Spannbackenhülse sowie die auswechselbare Halterung des Schaftes vorzugsweise rostfreier Stahl oder hochverdichtetes Titan verwendet. Für den Griff eignet sich jeder vollsterilisierbare Kunststoff, insbesondere Tetrafluoräthylen.

Aus einer Zeichnung Nr. 10958/9014 der Firma Robert Schröder, 5600 Wuppertal, vom März 1969 ist bereits ein Schraubendreher mit auswechselbarem Schrauberwerkzeug bekannt. Dabei wird ebenfalls eine federbelastete Schiebehülse mit Kugelgesperre zur Festlegung des Werkzeugschaftes verwendet. Jedoch ist dort weder eine Schraubenentnahmeeinrichtung mit Greiforgan für den Schraubenkopf noch ein bequem und sicher zu betätigender, zweigeteilter Handgriff vorgesehen.

Die Unterteilung eines Schraubendreher-Handgriffs ist an sich ebenfalls bereits aus der DE-OS 30 03 118 bekannt. Dort handelt es sich jedoch um einen in einem mittleren Handgriffteil fest verankerten gekrümmten Schraubendreher mit zwei an beiden Enden des Handgriffs drehbar gelagerten Kugeln, die eine Kreisbewegung des rückwärtigen Klingenendes um die Klingenachse ermöglichen. Diese bekannte Unterteilung des Handgriffs ist bei der erfindungsgemäßen Lösung nicht anwendbar.

Die Unteransprüche betreffen vorteilhafte Ausgestaltungen der Erfindung.

Anhand der Figuren wird ein bevorzugtes Ausführungsbeispiel der Erfindung näher erläutert. Es zeigen:

Fig. 1 eine Seitenansicht des Schraubendrehers bei zurückgezogener Spannhülse,

Fig. 2 einen Axialschnitt des Schraubendrehers bei vorgeschobener Spannhülse,

4/53

- 4 -

Zwecke, sondern ganz allgemein für diffizilere Verschraubungsvorgänge einsetzbar ist.

Die Unteransprüche 2 bis 4 betreffen vorteilhafte Ausgestaltungen der erfindungsgemäß verwendeten Spannbackenhülse und der angrenzenden Teile des Schraubendrehers.

Da das jeweils verwendete Werkzeug des Schraubendrehers meist nur für eine Art von Schraubenköpfen geeignet ist, ist gemäß den Ansprüchen 5 bis 8 eine auswechselbare Halterung des das Werkzeug tragenden Schaftes am Handgriff des Geräts vorgesehen. Durch das Auswechseln des Schaftes können nicht nur Werkzeuge für verschiedene Schraubenköpfe, sondern auch Gewindeschneider, ein Trokar, eine Raspel oder eine Feile mit einem dazugehörigen Schaft in das Gerät eingesetzt werden. Auch sind für verschiedene Operationstechniken verschieden lang bemessene Schäfte erforderlich, so daß es durch die Auswechselbarkeit des Schaftes ohne weiteres möglich ist, Werkzeuge mit unterschiedlich langen Schäften einzusetzen. Die Demontierbarkeit des erfindungsgemäßen Schraubendrehers ergibt überdies eine sehr gute Sterilisierbarkeit, was für die Anwendung bei einer Operation wichtig ist.

Ein sicheres und feinfühliges Einschrauben einer Schraube ist aber nicht nur von der Verwendung eines durchgängig von der Entnahme bis zum Festdrehen verwendbaren einzigen Gerätes, sondern auch von einer unter Operationsbedingungen, beispielsweise mit Operationshandschuh, einwandfreien und feinfühligen Betätigbarkeit des Schraubendrehers abhängig. Die Ansprüche 9 bis 11 sind daher auf eine zweckmäßige zweiteilige Ausgestaltung des Handgriffes gerichtet, die es ermöglicht, den rückwärtigen Griffteil fest in der Hand zu halten und den vorderen, dagegen drehbaren Griffteil mit Daumen und Zeigefinger feinfühlig zu drehen, ohne Reibungskräfte zwischen der Hand bzw. dem Handschuh und dem festliegenden rückwärtigen Griffteil hervorzurufen. Dadurch läßt sich der Schraubendreher mit Fingerspitzengefühl drehen und das auf die Schraube zu übertragende Drehmoment wird begrenzt, so daß ein Überdrehen der Schraube weitgehend vermieden wird. Bei herkömmlichen Schraubendrehern kann die Schraube mit der gesamten Kraft des Hand- oder Armgelenkes angezogen werden, was häufig zum Ausreißen der Schraube aus dem Knochen oder zur Beschädigung des Schraubenkopfes führt. Außerdem ist bei herkömmlichen Schraubendrehern mit nicht unterteiltem Handgriff nicht selten der Handschuh des Operateurs beschädigt worden, was entweder die Infektionsgefahr erhöht oder aber einen Handschuhwechsel erforderlich macht.

Im Hinblick auf Festigkeit und Sterilisierbarkeit werden als Materialien für das Werkzeug, die Spannhülse und die Spannbackenhülse sowie für die auswechselbare Halterung des Schaftes vorzugsweise rostfreier Stahl oder hochverdichtetes Titan verwendet. Für den Griff eignet sich jeder voll sterilisierbare Kunststoff, insbesondere Tetrafluoräthylen.

Anhand der Figuren wird ein bevorzugtes Ausführungsbeispiel der Erfindung näher erläutert. Es zeigen:

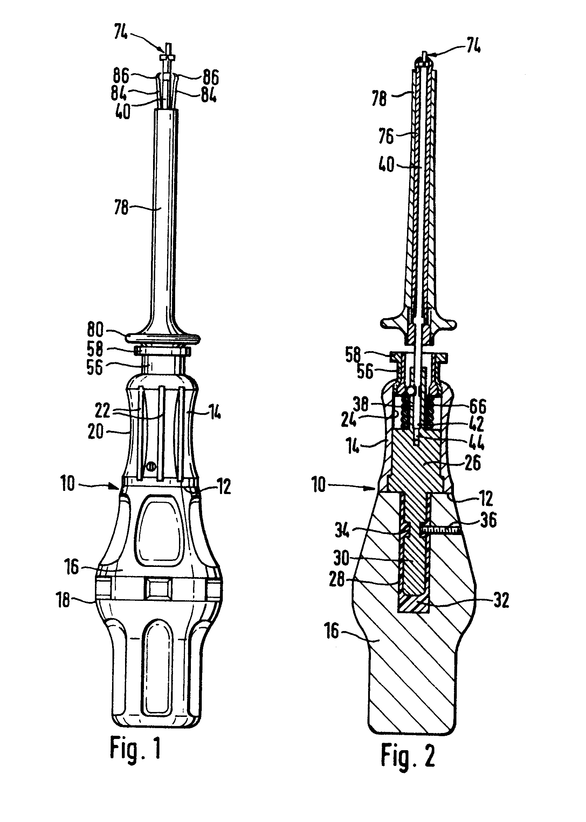

Fig. 1   eine Seitenansicht des Schraubendrehers bei zurückgezogener Spannhülse,

Fig. 2   einen Axialschnitt des Schraubendrehers bei vorgeschobener Spannhülse,

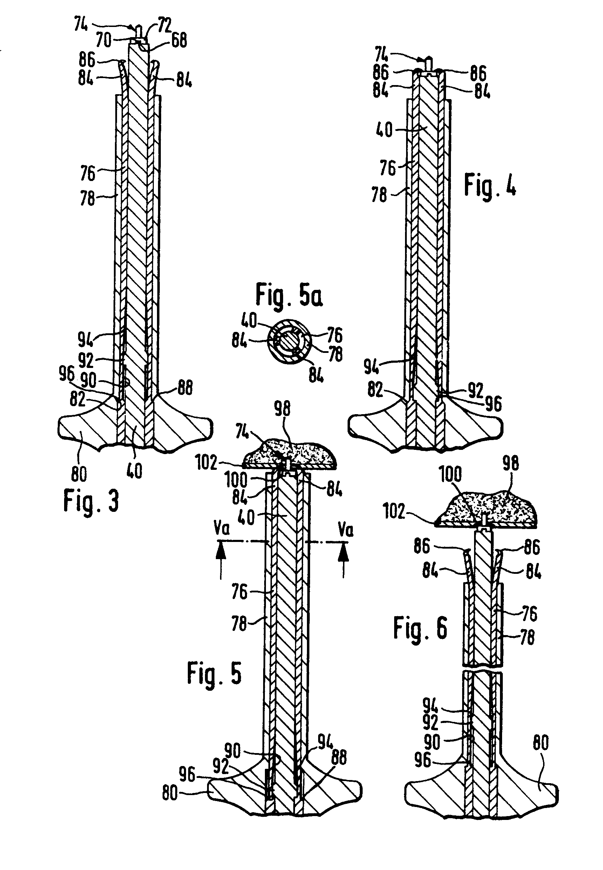

Fig. 3 bis 6    den  den vorderen Abschnitt des
          Schaftes, der Spannbackenhülse und der Spann-
          hülse umfassenden Abschnitt des Schraubendre-
          hers in vier aufeinanderfolgenden Stadien des
          Entnehmens und Einschraubens einer Knochenschraube,

Fig. 5a einen Schnitt längs der Linie Va-Va in Fig. 5
          sowie

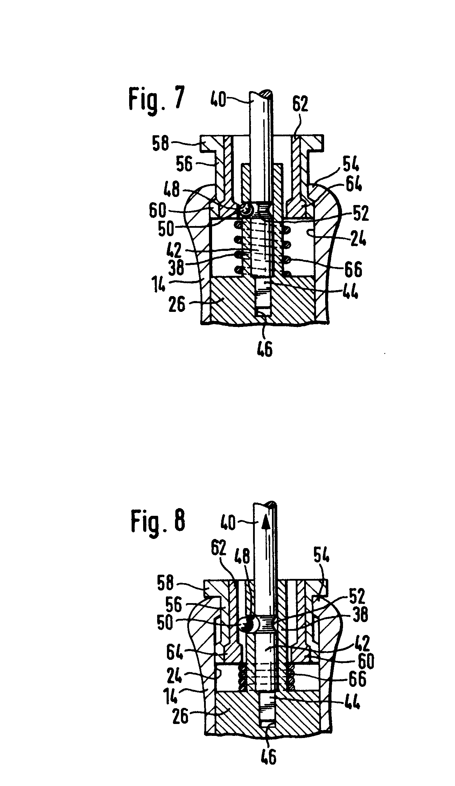

Fig. 7 und 8  das werkzeugseitige Ende des Handgriffs
          mit der Einrichtung zur auswechselbaren Halterung des Schaftes in zwei unterschiedlichen
          Betriebszuständen.

Der in den Figuren dargestellte Schraubendreher weist
einen allgemein mit 10 bezeichneten Handgriff auf, der
längs einer Radialebene 12 in zwei axial aneinandergrenzende Teile, nämlich einen vorderen Griffteil 14
und einen rückwärtigen Griffteil 16 unterteilt ist.
Der rückwärtige Griffteil 16 besitzt eine ausgebauchte
und sich der Handinnenfläche ergonomisch günstig anpassende Oberfläche 18, während der vordere Griffteil
14 eine leicht eingezogene Oberfläche 20 aufweist, auf
die Längsrippen 22 zum sicheren Drehen mittels der
Fingerspitzen von Daumen und Zeigefinger und ggf. Mittelfinger aufgesetzt sind. Der vordere Griffteil 15 weist
eine nach vorne offene Ausnehmung 24 auf, in die ein
Einsatzteil 26 fest eingefügt ist. Am Einsatzteil 26
sitzt ein nach rückwärts in eine Ausnehmung 28 des
rückwärtigen Griffteils 16 ragender Dorn 30, wobei
zwischen dem Dorn 30 und der Ausnehmung 28 ein gleitfähiges Lagermaterial 32 eingefügt ist. Der Dorn 30
weist eine Ringnut 34 auf, in die eine durch eine Bohrung des rückwärtigen Griffteils 16 eingesetzte Maden-

- 7 -

schraube 36 mit Spiel eingreift. Dadurch ist eine angemessene Drehbarkeit des vorderen Griffteils 14 gegenüber dem rückwärtigen Griffteil 16 gewährleistet.

Von der Mitte des Einsatzteils 26 steht ferner ein hohlzylindrischer Ständer 38 nach vorne, der über das offene vordere Ende des vorderen Griffteils 14 hinausragt. In den Ständer 38 ist das rückwärtige Ende 42 eines Schaftes 40 eingesetzt, wobei an diesem rückwärtigen Ende 42 ein Mehrkant 44 sitzt, der mit geringem Spiel in eine entsprechende Mehrkantfassung 46 des Einsatzteils 46 eingefügt ist. Dadurch dreht sich der Schaft 40 zwangsläufig mit dem vorderen Griffteil 14. Der Ständer 38 weist einen radialen Durchbruch 48 auf, in dem eine Kugel 50 radial beweglich gelagert ist. Bei vollständig in den Ständer 38 eingesetztem rückwärtigen Ende 42 des Schaftes 40 liegt eine Umfangsnut 52 des Schaftes 40 in gleicher Höhe wie der Durchbruch 48 mit der Kugel 50. Die Kugel 50 kann daher in die Nut 52 eindringen. An seinem vorderen Ende weist der vordere Griffteil 14 eine ringförmige Einziehung 54 auf, die an der Außenseite einer im wesentlichen zylindrischen Schiebehülse 56 anliegt. Die Schiebehülse 56 weist einen radial vorstehenden vorderen Anschlagring 58 und einen entsprechenden rückwärtigen Anschlagring 60 auf, so daß sie bezüglich des vorderen Griffteils 14 zwischen den Stellungen der Fig. 7 und der Fig. 8 axial verschiebbar ist. Die Schiebehülse 46 weist einen ihre Innenseite bildenden metallischen Einsatz 62 auf, der ebenfalls im wesentlichen zylindrisch geformt ist und an seinem inneren Ende eine ringförmige Verdickung 64 besitzt.

Eine Schraubendruckfeder 66 ist einerseits an der Vorderseite des Einsatzteils 26 und andererseits an der Verdickung 64 abgestützt und drückt die Schiebehülse 56 in die Stellung der Fig. 7. In dieser Stellung liegt die Verdickung 64 an der Kugel 50 an und drückt diese in die Nut 52. Dadurch ist der Schaft 40 am Ständer 38 verriegelt. Durch Zurückziehen der Schiebehülse 56 aus der Stellung der Fig. 7 in die Stellung der Fig. 8 entgegen der Kraft der Druckfeder 66, wozu der vordere Anschlagring 58 benutzt werden kann, gerät die Verdickung 64 außer Eingriff mit der Kugel 50, so daß diese aus der Nut 52 austreten und der Schaft 40 aus dem Ständer 38 herausgezogen werden kann. Anschließend läßt sich ein Schaft eines anderen Werkzeugs einsetzen, worauf die Schiebehülse 56 losgelassen wird und durch die Kraft der Druckfeder 66 wieder in die Stellung gemäß Fig. 7 zurückschnappt. Dadurch ist der Schaft des neuen Werkzeugs wieder sicher verriegelt. Die Einzelheiten der Schraubenentnahmeeinrichtung und des mit dieser zusammenwirkenden Werkzeugschaftes sind am besten aus den Fig. 3 bis 6 zu ersehen. Bei der dargestellten Ausführungsform ist das am vorderen Ende des Schaftes 40 sitzende Werkzeug als Schraubendreherklinge 68 zum Eingriff in den geradlinigen Längsschlitz 70 im Kopf 72 einer Knochenschraube 74 ausgebildet. In der Stellung der Fig. 3 befindet sich die den Schaft 40 umschließende und auf diesem gleitbar gelagerte Spannbackenhülse 76 und die die Spannbackenhülse 76 umfassende und gegen diese ebenfalls längsverschiebbare Spannhülse 78 in zurückgezogenem Zustand. Das Zurückziehen der Spannbackenhülse 76 und der Spannhülse 78 erfolgt mittels eines mit der Spannhülse 78 einstückigen Betätigungsringes 80, der von den Fingern der das Gerät führenden Hand erfaßt werden kann. Während die Spannhülse 78 im wesentlichen zylindrisch verläuft und lediglich nahe dem Betätigungsring 80 einen nach rückwärts weisenden Absatz 82 aufweist, geht die im wesentlichen ebenfalls zylindrische Spannbackenhülse 76 an ihrem vorderen, also werkzeugseitigen Ende in zwei diametral gegenüberliegende Spannbacken über, die einstückig mit der Spannbackenhülse 76 verbunden sind und in dem von der Spannhülse 78 unbelasteten Zustand gemäß Fig. 3 in der in dieser Figur dargestellten Weise durch Eigenelastizität leicht nach
außen federn. Am vorderen Ende weisen die Spannbacken
84 nach innen gebogene Greifer 86 auf.

Die Spannbackenhülse 76 weist an ihrem nahe dem Betätigungsring 80 verlaufenden Abschnitt an ihrer
Außenseite einen mit dem Absatz 82 der Spannhülse 78
zusammenwirkenden Absatz 88 auf, der das Zurückziehen
der Spannhülse 78 bezüglich der Spannbackenhülse 76
auf die in Fig. 3 dargestellte gegenseitige Stellung
begrenzt. Nahe diesem Absatz 88 ist auf der Innenseite
der Spannbackenhülse 76 eine rings um den Innenumfang
der Spannbackenhülse 76 verlaufende Aussparung 90 vorgesehen, in der ein ringförmiger Vorsprung 92 an der
Außenseite des Schaftes 40 längsverschiebbar zwischen
den Endanschlägen 94 und 96 verschiebbar ist. Die beiden Anschläge 94 und 96 begrenzen die Relativbewegung
zwischen dem Schaft 40 und der Spannbackenhülse 76
in beiden Richtungen.

Zum Entnehmen einer Knochenschraube 74 aus einem nicht
gezeigten Schraubensortiment wird zunächst die Klinge
68 in den Längsschlitz 70 der Knochenschraube eingesetzt, während sich Spannbackenhüle 76 und Spannhülse
78 in der in Fig. 3 gezeigten zurückgezogenen Stellung
befinden. Sodann wird mittels des Betätigungsrings 80
die Spannhülse 78 in die Stellung der Fig. 4 vorgeschoben. Sie nimmt dabei infolge der leicht ausgespreizten
Spannbacken 84 die Spannbackenhülse 76 mit nach vorne, bis der rückwärtige Anschlag 96 der Aussparung 90 am Vorsprung 92 des Schaftes 40 zur Anlage kommt. Bei weiterem Vorschieben der Spannhülse 78 werden die Spannbacken 84 nach innen gedrückt, wobei die Greifer 86 den Kopf 72 der Knochenschraube 74 hintergreifen, wie in Fig. 4 dargestellt. Nach dem vollständigen Vorschieben der Spannhülse 78 in die Stellung der Fig. 5, wodurch sich die Absätze 82 und 88 voneinander entfernen, ist die Knochenschraube 74 nunmehr im Zusammenwirken von Klinge 68 und Greifern 86 am Schraubendreher vollständig festgelegt. In diesem Zustand kann die Knochenschraube 74 in einen Knochen 98, ggf. durch eine Öffnung 100 einer am Knochen 98 festzulegenden Knochenplatte 102 hindurch, eingeschraubt werden, bis die Greifer 86 in leichte Anlage an der Außenseite der Knochenplatte 102 kommen. Zweckmäßigerweise kurz vor diesem Zeitpunkt wird der Betätigungsring 80 und somit die Spannhülse 78 in die Stellung der Fig. 3 zurückgezogen, wobei zunächst die Spannbacken 84 freigegeben werden und nach außen federn. Sobald die Absätze 82 und 88 zur gegenseitigen Anlage kommen, wird die Spannbackenhülse ebenfalls mit zurückgezogen, bis wieder der vordere Anschlag 94 der Aussparung 90 am Vorsprung 92 zur Anlage kommt, wie in Fig. 6 dargestellt. Dieser ganze Freigabevorgang der Knochenschraube 74 kann sehr schnell, also praktisch während des Einschraubvorganges durch Zurückziehen des Betätigungsringes 80 erfolgen und anschließend kann die Knochenschraube 74 vollständig festgezogen werden, wie in Fig. 6 gezeigt.

Während die stärker beanspruchten Teile des Schraubendrehers, wie Schaft 40, Werkzeug 68, Spannbackenhülse 76, Spannhülse 78, Einsatz 62, Kugel 50 und Einsatz 26 mit Ständer 38, wie oben erwähnt, vorzugsweise aus rostfreiem Stahl oder hochverdichtetem Titan hergestellt

- 1‌1 -

sind, bestehen die äußeren Teile des Handgriffes 10
und der Schieberhülse 56 vorzugsweise aus vollsterilisierbarem Kunststoff, beispielsweise Tetrafluoräthylen.

0222971

Dipl. Ing. Klaus Westphal

Dr. rer. nat. Bernd Mussgnug

Dr. rer. nat. Otto Buchner

P A T E N T A N W Ä L T E
European Patent Attorneys

Waldstrasse 33

D-7730 VS-VILLINGEN

Flossmannstrasse 30a

D-8000 MÜNCHEN 60

Telefon  07721-58007
Telegr.  Westbuch Villingen
Telex    5213177 webu d

Telefon  089-832446
Telegr.  Westbuch München
Telex    5213177 webu d
Telecop. 089-8344618
(CCITT2) attention webu

Patentanmeldung P 35 39 502.8-15

- Oskar Leibinger GmbH -

u.Z.: 971.31
-EP

PATENTANSPRÜCHE

1. Schraubendreher, insbesondere für chirurgische Zwecke, mit einem Handgriff, an dem ein Schaft befestigt ist, einem am Schaft sitzenden Werkzeug und einer Schraubenentnahmeeinrichtung, die ein Greiforgan für einen Schraubenkopf und eine mit dem Greiforgan zusammenwirkende, längs des Schaftes verschiebbare Spannhülse aufweist, wobei auf dem Schaft und innerhalb der Spannhülse das als Spannbackenhülse ausgebildete Greiforgan gleitend verschiebbar gelagert ist, an dessen werkzeugseitigem Ende zwei diametral gegenüberliegende, in von der Spannhülse unbelastetem Zustand vom Schaft weg elastisch nach außen federnde Spannbacken für den Schraubenkopf angeordnet sind, und wobei die Längsbewegung der Spannbackenhülse bezüglich des Schaftes in beiden Richtungen und die Längsbewegung der Spannhülse bezüglich der Spannbackenhülse in Richtung weg vom Werkzeug jeweils durch Anschläge begrenzt ist, dadurch gekennzeichnet, daß der Handgriff (10) axial in einen vorderen Griffteil (14) und einen rückwärtigen Griffteil (16) unterteilt ist, die gegenseitig drehbar aneinander festgelegt sind, daß zur auswechselbaren Halterung des Schaftes (40) am vorderen Griffteil (14) der Schaft (40) eine Nut (52) und der vordere Griffteil (14) ein mit der Nut (52) zusammenwirkendes Kugelgesperre (48, 50) aufweist, das durch eine in einer Ausnehmung (24) des vorderen Griffteils (14) axial verschiebbar gelagerte Schiebehülse (56) aus- und einklinkbar ist, wobei die Schiebehülse (56) durch eine Druck-Postscheckkonto Karlsruhe 76979-754  Bankkonto: Deutsche Bank AG Villingen (BLZ 69470039) 146332

feder (66) in Richtung zum Werkzeug (68) hin belastet ist, und daß die Kugel (50) des Kugelgesperres (48, 50) in einem radialen Durchbruch (48) eines den Schaft (40) aufnehmenden, am vorderen Griffteil (14) sitzenden Ständers (38) angeordnet ist.

2. Schraubendreher nach Anspruch 1, dadurch gekennzeichnet, daß in den vorderen Griffteil (14) ein Einsatzteil (26) fest eingefügt ist, der einen nach rückwärts weisenden und in einer Ausnehmung (28) des rückwärtigen Griffteils (16) drehbar gelagerten Dorn (30) aufweist.

3. Schraubendreher nach Anspruch 2, dadurch gekennzeichnet, daß zwischen Dorn (30) und Ausnehmung (28) ein gleitfähiges Lagermaterial (32) eingefügt ist.

4. Schraubendreher nach Anspruch 2 oder 3, dadurch gekennzeichnet, daß der Dorn (30) eine Ringnut (34) aufweist, in die eine durch eine Gewindebohrung des rückwärtigen Griffteils (16) eingesetzte Madenschraube (36) mit Spiel eingreift.

5. Schraubendreher nach einem der vorangehenden Ansprüche, dadurch gekennzeichnet, daß der rückwärtige Griffteil (16) zur festen Einfügung in eine greifende Hand ausgebaucht ist und daß der vordere Griffteil (14) zur Drehung mittels Fingerspitzen eine eingezogene Form besitzt.

6. Schraubendreher nach Anspruch 5, dadurch gekennzeichnet, daß der vordere Griffteil (14) mit Längsrippen (22) versehen ist.

Fig. 1

Fig. 2

0222971

Fig. 3

Fig. 5a

Fig. 4

Fig. 5

Fig. 6

Fig. 7

58
56
48
60
50
42
38
14
26

40    62

54
64
52
24
66
44

46

Fig. 8

40

62  48

58
56
50

64
24
14
26

54

52
38

42
60
66
44

46

Europäisches
Patentamt

**EUROPÄISCHER RECHERCHENBERICHT**

Nummer der Anmeldung

| | **EINSCHLÄGIGE DOKUMENTE** | | EP 86109433.2 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 324 756 (HILTI) <br> * Fig. 1,2 * <br> -- | 1 | B 25 B 23/08 <br> A 61 B 17/56 |
| A | US - A - 3 498 351 (G.Z. EDWARDS) <br> * Fig. 3 * <br> -- | 1 | |
| A | US - A - 4 480 668 (LIN) <br> * Fig. 1, Pos. 3 * <br> -- | 1 | |
| A | US - A - 4 417 611 (KIM) <br> * Fig. 1 * <br> ---- | 1 | |

| RECHERCHIERTE SACHGEBIETE (Int. Cl.4) |
|---|
| B 25 B 15/00 |
| B 25 B 23/00 |
| A 61 B 17/00 |
| B 25 G 1/00 |

Der vorliegende Recherchenbericht wurde für alle Patentansprüche erstellt.

| Recherchenort | Abschlußdatum der Recherche | Prüfer |
|---|---|---|
| WIEN | 12-02-1987 | BENCZE |